# EUROPÄISCHE PATENTSCHRIFT

(11) **EP 0 656 346 B1**
(45) Veröffentlichungstag und Bekanntmachung des Hinweises auf die Patenterteilung: **05.02.1997**
(21) Anmeldenummer: 93119406.2
(22) Anmeldetag: 02.12.1993
(51) Int. Cl.: C07C 233/36, C07C 233/38, C07C 229/16, C07C 215/40, C11D 1/90

(54) **Verfahren zur Herstellung hochkonzentrierter fliessfähiger wässriger Lösungen von Betainen**
Method for the preparation of highly concentrated flowable aqueous solutions of betaines
Procédé pour la préparation des solutions des betaines qui sont fluxables et très concentrés

(43) Veröffentlichungstag der Anmeldung: 07.06.1995
(73) Patentinhaber: Witco Surfactants GmbH, D-36396 Steinau an der Strasse (DE)
(72) Erfinder: Hamann, Ingo, Dr., D-63619 Bad Orb (DE); Köhle, Hans-Jürgen, Dr., D-36381 Schlüchtern (DE); Stark, Klaus, D-63589 Linsengericht 4 (DE); Wehner, Winfried, Dr., D-36119 Neuhof (DE)

(56) Entgegenhaltungen:
- EP-A- 0 243 619
- EP-A- 0 302 329
- EP-A- 0 353 580
- EP-A- 0 560 114
- WO-A-91/08193

## Beschreibung

Die Erfindung betrifft ein Verfahren zur Herstellung hochkonzentrierter fließfähiger wässriger Lösungen von Betainen, welche Feststoffgehalte von mehr als 40 Gew.-%, vorzugsweise mehr als 50 Gew.-%, enthalten.

Betaine haben sich in den letzten Jahren in der kosmetischen Industrie als fester Bestandteil von Rezepturen, insbesondere für die Haar- und Körperreinigung, etabliert. Sie haben die Fähigkeit, einen dichten und sahnigen Schaum auszubilden, welcher auch in Gegenwart anderer Tenside, Seifen und Additive über einen langen Zeitraum stabil bleibt, verbunden mit anerkannt guten Reinigungseigenschaften ohne irgendwelche irritierenden Nebenwirkungen, selbst für empfindliche Haut.

Die Herstellung von Betainen wird in der einschlägigen Patent- und Fachliteratur ausführlich beschrieben (US-PS 3 225 074). Im allgemeinen werden dabei tertiäre Aminstickstoffatome enthaltende Verbindungen mit ω-Halogencarbonsäuren oder deren Salze in wässrigen oder wasserhaltigen Medien umgesetzt. Als tertiäre Aminstickstoffatome enthaltende Verbindungen werden insbesondere Fettsäureamide der allgemeinen Formel (II)

R³-CONH-(CH₂)ₘ-NR⁴R⁵ (II)

eingesetzt, worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedenen Alkylreste mit 1 - 4 C-Atomen bedeuten und m = 1 - 3 sein kann.

Der Alkylrest R³ kann sich hierbei von den natürlichen oder synthetischen Fettsäuren mit 6 - 20 C-Atomen, vorzugsweise von den natürlichen pflanzlichen oder tierischen Fettsäuren mit 8 - 18 C-Atomen ableiten sowie deren natürlich vorkommenden speziell eingestellten Mischungen miteinander oder untereinander.

Als Fettsäuren kommen beispielsweise Capronsäure, Caprylsäure, Caprinsäure, Laurinsäure, Palmitinsäure, Stearinsäure, Linolsäure, Linolensäure, Ricinolsäure in Betracht.

Bevorzugt werden die natürlich vorkommenden Fettsäuremischungen mit einer Kettenlänge von 8 - 18 C-Atomen, wie Kokosfettsäure oder Palmkernfettsäure, welche gegebenenfalls durch geeignete Hydrierungsmethoden gehärtet werden können.

Mit diesen Fettsäuren bzw. Fettsäuremischungen wird durch übliche Kondensationsreaktion bei 140 - 200 °C mit Aminen der allgemeinen Formel (III)

H₂N-(CH₂)ₘ-NR⁴R⁵ (III)

- in welcher R⁴ und R⁵ und m die in der Formel (II) genannte Bedeutung haben - zu den Fettsäureamiden mit tertiären Stickstoffatomen der allgemeinen Formel (II) umgesetzt.

Die anschließende Quaternierungsreaktion zu Betainen der Formel (IV)

R³-CONH-(CH₂)ₘ-N⁺R⁴R⁵(CH₂)_{y}COO⁻ (IV)

worin R³, R⁴, R⁵ und m die gleiche Bedeutung wie in den Formeln (II) und (III) haben und y = 1, 2, 3 sein kann, kann nach den literaturbekannten Verfahren durchgeführt werden.

Dem Fettsäureamid der Formel (II) werden dabei in der Regel in wässrigem Medium ω-Halogenalkylcarbonsäuren, vorzugsweise das Natriumsalz der Chloressigsäure, zugesetzt und bei einer mehrstündigen Reaktion bei ca. 80 - 100 °C die Quaternierung vollzogen. Je nach eingesetzter Fettsäure bzw. Fettsäuremischung muß zum Erhalt der Rührfähigkeit bei fortschreitender Reaktion eine Mindestmenge an Wasser vorhanden sein. Die handelsübliche Konzentration an Betaingehalt der auf diese Weise hergestellten Lösungen liegt daher bei etwa 30 Gew.-% oder darunter.

Zur Einsparung von Lagerungs- und Transportkosten sowie aus formulierungstechnischen Gründen bei der Weiterverarbeitung war aber in vielen Fällen eine höhere Konzentration dringend erwünscht.

In der Vergangenheit sind daher eine Reihe von Verfahren vorgeschlagen worden, welche dieses Problem lösen sollten. So ist aus der DE-PS 3 613 944 ein Verfahren bekannt, bei dem die Quaternierung in einem organischen polaren Lösungsmittel mit einem Wasseranteil von 20 Gew.-% durchgeführt und danach das Lösungsmittel ganz oder teilweise destillativ entfernt und dann wieder mit einem anwendungstechnisch brauchbaren Lösungsmittel auf die gewünschte Konzentration eingestellt wird.

Abgesehen davon, daß das Verfahren technisch aufwendig und kostenintensiv ist, sind organische Lösungsmittel bei der Weiterverarbeitung in kosmetischen Rezepturen vielfach unerwünscht.

Das Verfahren gemäß DE-PS 3 726 322 kommt zwar ohne organische Lösungsmittel aus, jedoch muß die für die Quaternierungsreaktion erforderliche Wassermenge durch Destillation wieder aus dem Reaktionsprodukt entfernt werden, wobei vor oder nach der Einstellung auf die gewünschte Konzentration der pH-Wert der Lösung durch relativ hohe Säuremengen auf hautuntypische Werte von 1 - 4,5 eingestellt werden muß.

Gemäß EP-A-0 353 580 werden dem Reaktionsgemisch aus Fettsäureamid und Halogenalkylcarbonsäure vor oder während der Quaternierungsreaktion oder der erhaltenen Lösung des Betains nichtionogene, wasserlösliche Tenside in solchen Mengen zugesetzt, daß die fertige Lösung 3 - 20 Gew.-% wasserlösliche Tenside enthält.

Als nichtionogene Tenside werden Polyoxyethylenether eingesetzt, welche für eine ausreichende Wasserlöslichkeit 10 - 250 Oxyethyleneinheiten enthalten müssen.

Polyoxyethylenether mit höheren Anteilen an Oxyethyleneinheiten haben sich hinsichtlich ihrer biologischen Abbaubarkeit aber als nicht unproblematisch erwiesen.

Es bestand daher weiterhin ein Bedarf an hochkonzentrierten, fließ- und pumpfähigen, wässrigen Lösungen von Betainen, welche frei von niedrigen Alkoholen wie Methanol, Ethanol, Propanol oder Isopropanol sind.

Überraschenderweise wurde nun gefunden, daß man fließ- und pumpfähige 30 bis 50 Gew.-prozentige Lösungen von Betainen aus dem Reaktionsgemisch aus Fettsäureamid und ω-Halogenalkylcarbonsäure herstellen kann, insbesondere wenn man dem Fettsäureamid der allgemeinen Formel (II) und dem Natriumsalz der Chloressigsäure vor oder während der Quaternierungsreaktion 0,5 - 5 Gew.-%, vorzugsweise 2 - 4 Gew.-%, bezogen auf die Gesamtmischung, Verbindungen der allgemeinen Formel (I) 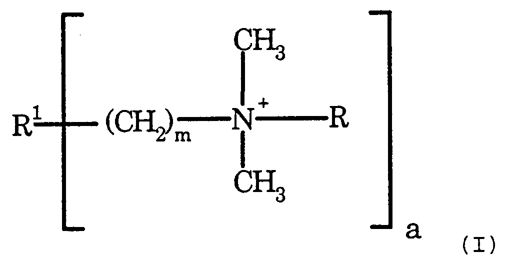
worin R = CH₃-, -CH₂COO⁻ K⁺, mit K⁺ = Ammonium oder Alkalimetallkation; R¹ = -N(R)₂ mit R = -CH₂COO⁻ K⁺; R¹ = OH mit R = CH₃ und m = 2; R¹ = -NHCO-(CHR²)ₓ-CH₃ mit R² = H oder ein oder mehrere statistisch über die Kette verteilte C₁- bis C₃-Alkylgruppen und/oder OH-Gruppen und x = 0 bis 8, vorzugsweise 0 bis 3; R¹ = -NHCO-(CHR²)_{y}-CONH- mit R² = H oder ein oder mehrere statistisch über die Kette verteilte C₁- bis C₃-Alkylgruppen und mit a = 2 und y = 0 bis 8, vorzugsweise 2 bis 6, zusetzt. Die Mischung enthält weiterhin 5 - 8 Gew.-% Natriumchlorid und ad 100 Gew.-% Wasser.

Die erfindungsgemäßen Verbindungen der allgemeinen Formel I sind herstellbar durch Umsetzung von Monocarbonsäuren oder deren Derivate Formel V

CH₃-(CHR²)ₓ-COOR⁶ (V)

und/oder Dicarbonsäuren oder deren Derivate der allgemeinen Formel VI

R⁶OOC-(CHR²)ₓ-COOR⁷ (VI)

worin R² = H oder ein oder mehrere statistisch über die Kette verteilte C₁- bis C₃-Alkylgruppen und/oder OH-Gruppen, R⁶, R⁷ unabhängig voneinander H oder kurzkettige Alkylreste mit vorzugsweise 1 bis 3 C-Atomen, x = 0 bis 8, vorzugsweise 0 bis 1, und y = 0 bis 8, vorzugsweise 2 bis 6, sind, mit Aminen der allgemeinen Formel VII 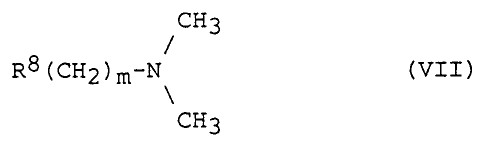
worin m = 1 bis 3 und R⁸ eine primäre oder sekundäre Aminogruppe oder eine Hydroxylgruppe ist und weitere Quaternierung mit ω-Halogenalkylcarbonsäuren, vorzugsweise dem Natriumsalz der Chloressigsäure nach an sich bekannten Verfahren durch mehrstündige Reaktion in wässrigem Medium bei ca. 80 - 100 °C.

Als Monocarbonsäuren oder Dicarbonsäuren werden erfindungsgemäß die Verbindungen der jeweiligen homologen Reihen eingesetzt, welche gegebenenfalls Alkyl- und/oder OH-Substituenten enthalten können, wie Essigsäure, Propionsäure, Buttersäuren, Valeriansäuren, Capronsäuren, Caprylsäuren, Caprinsäuren, Milchsäure, β-Hydroxy-propionsäure, γ-Hydroxyvaleriansäure, Oxalsäure, Malonsäure, Bernsteinsäure, Glutarsäure, Adipinsäure, Pimelinsäure, Suberinsäure, Azelainsäure, Sebazinsäure, Äpfelsäure, Tartronsäure, Weinsäure.

Diese Säuren können in Mischung sowohl miteinander als auch untereinander oder in Form ihrer Ester wie insbesondere ihrer Methylester oder Ethylester eingesetzt werden.

Die erfindungsgemäßen Verbindungen der allgemeinen Formel (I) sind herstellbar durch Quaternierungsreaktion der Verbindungen der Formel (VII) durch mehrstündige Umsetzung in wässrigem Medium bei 80 - 100 °C nach an sich bekannten Verfahren mit ω-Halogenalkylcarbonsäuren, vorzugsweise dem Natriumsalz der Chloressigsäure oder kurzkettige Dialkylphosphate, Dialkylsulfate, Halogenkohlenwasserstoffe wie insbesondere Dimethylsulfat, Diethylsulfat, Dimethylphosphat, Diethylphosphat, Methylchlorid.

Ohne weitere Aufkonzentrierungsarbeiten, allein durch entsprechende Bemessung des Wasseranteils bei der Quaternierungsreaktion, werden wässrige Lösungen von Betainen erhalten, welche auch ohne Mitverwendung von kurzkettigen, monofunktionellen Alkoholen ohne weiteres fließ- und pumpfähig sind.

Bereits mit Anteilen von ≤5 Gew.-% an Verbindungen der allgemeinen Formel (I) können fließ- und pumpfähige Mischungen erhalten werden, welche Betaine auf Basis der Fettsäureamide der Formel (II) in Mengen von >30, teilweise >50 Gew.-%, bezogen auf Trockensubstanz, enthalten.

Da der Zusatz dieser Verbindungen bei der weiteren Verarbeitung zur Herstellung von Haar- und Körperreinigungsmitteln nicht stört, sie toxikologisch unbedenklich sind und biologisch problemlos abgebaut werden, können, falls im Einzelfalle gewünscht, auch höhere Anteile mitverwendet werden.

Der verfahrensbedingte Anteil an NaCl in der Betainlösung kann in der Regel in der Lösung verbleiben, da zur Viskositätseinstellung von Shampoos oder Duschgelen der Zusatz von Elektrolytsalzen gängige Praxis ist.

Die erfindungsgemäß hergestellten hochkonzentrierten Betainlösungen sind über einen relativ weiten Temperaturbereich stabile homogene und klare Mischungen, welche sich für die weitere Verarbeitung komplikationslos auf die gewünschte Anwendungskonzentration verdünnen und compoundieren lassen.

### Analysenmethoden

### Trockensubstanz:

Die Trockensubstanz wird durch Trocknen des Materials bei 105 °C bis zur Gewichtskonstanz bestimmt. Diese Werte werden bestimmt nach den Einheitsmethoden der Deutschen Gesellschaft für Fettchemie (DGF): B-II.

### Säurezahl (SZ):

Die Säurezahl ist ein Maß für die in Fetten und technischen Fettsäuren enthaltenen freien Säuren. Sie gibt die Anzahl Milligramm Kaliumhydroxid an, die notwendig ist, um 1 Gramm Substanz oder technische Fettsäuren zu neutralisieren (mg KOH/g). Diese Werte werden bestimmt nach den Einheitsmethoden der Deutschen Gesellschaft für Fettchemie (DGF): D-IV 2a.

### Esterzahl (EZ):

Die Esterzahl ist ein Maß für die in Fetten und technischen Fettsäuren enthaltenen Ester. Sie gibt die Anzahl Milligramm Kaliumhydroxid an, die notwendig ist, um 1 Gramm Substanz oder technische Fettsäuren zu verseifen (mg KOH/g). Diese Werte werden bestimmt nach den Einheitsmethoden der Deutschen Gesellschaft für Fettchemie (DGF): C-V 4.

### Gesamtaminzahl (GAZ), Tertiäraminzahl (TAZ):

Die Gesamtaminzahl gibt die Anzahl Milligramm Kaliumhydroxid an, die der Gesamtbasizität von 1 Gramm der Aminverbindung äquivalent sind (mg KOH/g).

Die Tertiäraminzahl gibt die Anzahl Milligramm Kaliumhydroxid an, die der Tertiäraminbasizität von 1 Gramm der Aminverbindung äquivalent sind (mg KOH/g).

Die Werte werden bestimmt nach American Oil Chemists Society (A.O.C.S.) Official Method Tf 2a-64.

### Natriumchlorid:

Der Gehalt an Natriumchlorid wird potentiometrisch gegen eine Silbernitrat-Maßlösung ermittelt. Als Elektrode wird eine kombinierte Silberchlorid-Elektrode verwendet. Die Werte werden bestimmt nach den Einheitsmethoden der Deutschen Gesellschaft für Fettchemie (DGF): H-III 9.

### Beispiele

### Beispiel 1:

### Herstellung des Aminamids:

98,0 kg gehärtetes Kokosöl werden in einem Reaktor mit Rührer, Thermometer und Destillationsaufsatz unter Inertgasatmosphäre mit 56,8 kg Dimethylaminopropylamin versetzt und auf 150 - 160 °C erwärmt und unter Rückfluß gekocht. Nach Beendigung der Amidierung (Esterzahl <10 mg KOH/g) wird der Aminüberschuß bei dieser Temperatur im Vakuum abdestilliert. Die Destillation ist vollständig, wenn die Differenz aus Gesamtaminzahl und Tertiäraminzahl geringer als 3 mg KOH/g ist. Das erhaltene Aminamid hat eine GAZ von 170,6 mg KOH/g, eine TAZ von 168,6 mg KOH/g und eine Esterzahl von 2,8 mg KOH/g.

### Beispiel 2:

97,3 g Monochloressigsäure werden unter Kühlung in einem 1 l Kolben mit Rührer, Innenthermometer und pH-Elektrode mit 504 g Wasser verdünnt und mit 82 g Natriumhydroxid-Lösung (50 %) vorsichtig neutralisiert. Nach der Neutralisation wird die Natriumchloracetat-Mischung mit 20,9 g Cholinchlorid versetzt und auf 70 - 80 °C erhitzt. Nach Zusatz von 320 g des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 7,7 g Natriumhydroxid (als 50 prozentige Lösung) benötigt. Nach ca. 8 h Reaktionszeit ist die Alkylierung beendet. Man läßt die Betain-Mischung auf 50 °C abkühlen und stellt den pH-Wert mit 7,5 g 50prozentiger Citronensäure-Lösung auf 5,3 ein.

Das Endprodukt ist eine klare Flüssigkeit mit einer Viskosität von 130 mPas bei 20 °C, einem Trockenrückstand von 45,6 % und einem Natriumchlorid-Gehalt von 6,8 %.

### Beispiel 3:

97,3 g Monochloressigsäure werden unter Kühlung in einem 1 l Kolben mit Rührer, Innenthermometer und pH-Elektrode mit 510 g Wasser verdünnt und mit 82 g Natriumhydroxid-Lösung (50 %) vorsichtig neutralisiert. Nach der Neutralisation wird die Natriumchloracetat-Mischung mit 26,2 g Cholinchlorid versetzt und auf 70 - 80 °C erhitzt. Nach Zusatz von 320 g des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 2,3 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 5,6 g 50prozentiger Citronensäure-Lösung auf 5,4 ein.

Das Endprodukt ist eine klare Flüssigkeit mit einer Viskosität von 90 mPas bei 20 °C, einem Trockenrückstand von 45,6 % und einem Natriumchlorid-Gehalt von 6,8 %.

### Beispiel 4:

180,2 g (3 mol) Essigsäure und 400 g (3,9 mol) Dimethylaminopropylamin werden in einem 2 l Dreihalskolben mit Rührer, Rückflußkühler und Inertgaszuführung unter Stickstoffatmosphäre auf 160 °C erhitzt. Nach Beendigung der Amidierung (Säurezahl <30 mg KOH/g) wird der Aminüberschuß bei dieser Temperatur im Vakuum abdestilliert. Das erhaltene Aminamid hat eine GAZ von 378 mg KOH/g, eine TAZ von 366 mg KOH/g und eine Säurezahl von 3,1 mg KOH/g.

### Beispiel 5:

98 g Monochloressigsäure werden unter Kühlung in einem 1 l Kolben mit Rührer, Innenthermometer und pH-Elektrode mit 204 g Wasser verdünnt und mit 83 g Natriumhydroxid-Lösung (50 %) vorsichtig neutralisiert. Nach der Neutralisation wird die Natriumchloracetat-Mischung auf 70 - 80 °C erhitzt. Nach Zusatz von 144 g des Essigsäure-Aminamids aus Beispiel 4 wird das Reaktionsgemisch bei 80 - 90 °C gerührt. Dabei wird der pH-Wert zwischen 8 und 8,5 gehalten. Hierzu werden insgesamt weitere 5 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 7 g 50 prozentiger Citronensäure-Lösung auf 5,5 ein.

Die so erhaltene Essigsäurebetain-Lösung hat einen Trockenrückstand von 50,3 %.

### Beispiel 6:

97,3 g Monochloressigsäure werden unter Kühlung in einem 1 l Kolben mit Rührer, Innenthermometer und pH-Elektrode mit 497 g Wasser verdünnt und mit 82 g Natriumhydroxid-Lösung (50 %) vorsichtig neutralisiert. Nach der Neutralisation wird die Natriumchloracetat-Mischung mit 31,4 g Essigsäurebetain aus Beispiel 5 versetzt und auf 70 - 80 °C erhitzt. Nach Zusatz von 320 g des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 5,3 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 7,1 g 50prozentiger Citronensäure-Lösung auf 5,3 ein.

Das Endprodukt ist eine klare Flüssigkeit mit einer Viskosität von 128 mPas bei 20 °C, einem Trockenrückstand von 45,1 % und einem Natriumchlorid-Gehalt von 6,2 %.

### Beispiel 7:

104,5 g Monochloressigsäure werden unter Kühlung in einem 1 l Kolben mit Rührer, Innenthermometer und pH-Elektrode mit 505 g Wasser verdünnt und mit 87 g Natriumhydroxid-Lösung (50 %) vorsichtig neutralisiert. Nach der Neutralisation wird die Natriumchloracetat-Mischung mit 11 g Essigsäure-Aminamid aus Beispiel 4 versetzt und auf 70 - 80 °C erhitzt. Nach Zusatz von 320 g des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 8,1 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 6,0 g 50prozentiger Citronensäure-Lösung auf 5,3 ein.

Das Endprodukt ist eine klare Flüssigkeit mit einer Viskosität von 156 mPas bei 20 °C, einem Trockenrückstand von 44,6 % und einem Natriumchlorid-Gehalt von 6,35 %.

### Beispiel 8:

177 g (1,5 mol) Bernsteinsäure und 400 g (3,9 mol) Dimethylaminopropylamin werden in einem 2 l Dreihalskolben mit Rührer, Rückflußkühler und Inertgaszuführung unter Stickstoffatmosphäre auf 160 °C erhitzt. Nach Beendigung der Amidierung (Säurezahl <30 mg KOH/g) wird der Aminüberschuß bei dieser Temperatur im Vakuum abdestilliert. Das erhaltene Aminamid hat eine GAZ von 385 mg KOH/g, eine TAZ von 379 mg KOH/g und eine Säurezahl von 6,7 mg KOH/g.

### Beispiel 9:

98 g Monochloressigsäure werden unter Kühlung in einem 1 l Kolben mit Rührer, Innenthermometer und pH-Elektrode mit 205 g Wasser verdünnt und mit 82 g Natriumhydroxid-Lösung (50 %) vorsichtig neutralisiert. Nach der Neutralisation wird die Natriumchloracetat-Mischung auf 70 - 80 °C erhitzt. Nach Zusatz von 145 g des Bernsteinsäure-Aminamids aus Beispiel 8 wird das Reaktionsgemisch bei 80 - 90 °C gerührt. Dabei wird der pH-Wert zwischen 8 und 8,5 gehalten. Hierzu werden insgesamt weitere 23 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 7,5 g 50prozentiger Citronensäure-Lösung auf 5,6 ein. Die so erhaltene Bernsteinsäurebetain-Lösung hat einen Trockenrückstand von 50,1 %.

### Beispiel 10:

97,3 g Monochloressigsäure werden unter Kühlung in einem 1 l Kolben mit Rührer, Innenthermometer und pH-Elektrode mit 500 g Wasser verdünnt und mit 82 g Natriumhydroxid-Lösung (50 %) vorsichtig neutralisiert. Nach der Neutralisation wird die Natriumchloracetat-Mischung mit 23,8 g Bernsteinsäure-Betain aus Beispiel 9 versetzt und auf 70 - 80 °C erhitzt. Nach Zusatz von 320 g des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 6,7 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 7,5 g 50prozentiger Citronensäure-Lösung auf 5,5 ein.

Das Endprodukt ist eine klare Flüssigkeit mit einer Viskosität von 112 mPas bei 20 °C, einem Trockenrückstand von 44,7 % und einem Natriumchlorid-Gehalt von 6,2 %.

### Beispiel 11:

120,2 g Monochloressigsäure-Lösung (80 %) werden unter Kühlung in einem 1 l Kolben mit Rührer, Innenthermometer und pH-Elektrode mit 81 g Natriumhydroxid-Lösung (50 %) vorsichtig neutralisiert. Nach der Neutralisation wird die Natriumchloracetat-Mischung mit 34,6 g Dimethylaminopropylamin versetzt und auf 70 - 80 °C erhitzt. Dabei wird der pH-Wert zwischen 8 und 8,5 gehalten. Hierzu werden insgesamt weitere 55,4 g Natriumhydroxid (als 50prozentige Lösung) benötigt. Nach ca. 1,5 h Reaktionszeit ist die Alkylierung beendet. Man läßt die Betain-Mischung auf 50 °C abkühlen und stellt den pH-Wert mit 11,2 g 50prozentiger Citronensäure-Lösung auf 5,6 ein.

Das Endprodukt ist eine leicht trübe Flüssigkeit mit einem Trockenrückstand von 58,9 % und einem Natriumchlorid-Gehalt von 11,45 %.

### Beispiel 12:

97,3 g Monochloressigsäure werden unter Kühlung in einem 1 l Kolben mit Rührer, Innenthermometer und pH-Elektrode mit 500 g Wasser verdünnt und mit 82,3 g Natriumhydroxid-Lösung (50 %) vorsichtig neutralisiert. Nach der Neutralisation wird die Natriumchloracetat-Mischung mit 17,8 g des Betains aus Beispiel 11 versetzt und auf 70 - 80 °C erhitzt. Nach Zusatz von 320 g des Aminamids aus Beispiel 1 wird das Reaktionsgemisch bei 80 - 90 °C gerührt. Dabei wird der pH-Wert zwischen 8 und 8,5 gehalten. Hierzu werden insgesamt weitere 6,7 g Natriumhydroxid (als 50prozentige Lösung) benötigt. Nach ca. 8 h Reaktionszeit ist die Alkylierung beendet. Man läßt die Betain-Mischung auf 50 °C abkühlen und stellt den pH-Wert mit 9,4 g 50prozentiger Citronensäure-Lösung auf 5,9 ein.

Das Endprodukt ist eine klare Flüssigkeit mit einer Viskosität von 107 mPas bei 20 °C, einem Trockenrückstand von 44,0 % und einem Natriumchlorid-Gehalt von 6,1 %.

## Patentansprüche

1. Hochkonzentrierte Betaine, enthaltend 30 - 50 Gew.-% einer Verbindung der allgemeinen Formel (IV)
R³-CONH-(CH₂)ₘ-N⁺R⁴R⁵(CH₂)_{y}COO⁻ (IV)
worin R³ der Alkylrest einer Fettsäure, welcher gegebenenfalls verzweigt, gegebenenfalls Mehrfachbindungen, gegebenenfalls Hydroxylgruppen enthalten kann, R⁴ und R⁵ gleich oder verschiedene Alkylreste mit 1 - 4 C-Atomen bedeuten und m = 1 - 3 sein kann, 0,5 bis 5 Gew.-% mindestens einer Verbindung der allgemeinen Formel (I) 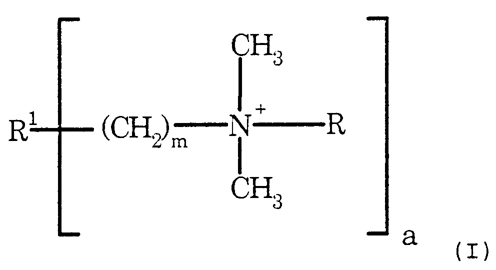 mit R = CH₃-, -CH₂COO⁻ K⁺, mit K⁺ = Ammonium oder Alkalimetallkation, a = 1-2, m = 1-3,
R¹ = -N(R)₂ mit R = -CH₂COO⁻ K⁺,
R¹ = OH mit R = CH₃ und m = 2,
R¹ = -NHCO-(CHR²)ₓ-CH₃ mit R² = H oder ein oder mehrere statistisch über die Kette verteilte C₁- bis C₃- Alkylgruppen und/oder OH-Gruppen und x = 0 bis 8,
R¹ = -NHCO-(CHR²)_{y}-CONH- mit R² = H oder ein oder mehrere statistisch über die Kette verteilte C₁- bis C₃-Alkylgruppen und mit a = 2 und y = 0 bis 8,
5 - 8 Gew.-% Natriumchlorid und ad 100 Gew.-% Wasser.

2. Hochkonzentrierte Betaine gemäß Anspruch 1, enthaltend 2 bis 4 Gew.-% der Verbindung der allgemeinen Formel (I) mit R¹ = -N(CH₂COO⁻ K⁺)₂, R = -CH₂COO⁻ K⁺ und m = 3.

3. Hochkonzentrierte Betaine gemäß Anspruch 1, enthaltend 2 bis 4 Gew.-% der Verbindung der allgemeinen Formel (I) mit R¹ = -NHCO-CH₃ und R = -CH₂COO⁻ K⁺ und m = 3.

4. Hochkonzentrierte Betaine gemäß Anspruch 1, enthaltend 2 bis 4 Gew.-% der Verbindung der allgemeinen Formel (I) mit R¹ = -NHCO-(CH₂)₄-CONH- und m = 3.

5. Verfahren zur Herstellung hochkonzentrierter fließfähiger wässriger Lösungen von Betainen durch Quaternierung von tertiärem Aminstickstoff enthaltenden Verbindungen mit Halogencarbonsäuren, dadurch gekennzeichnet, daß der Reaktionsmischung vor oder während der Quatenierungsreaktion mindestens eine der Verbindungen der allgemeinen Formel (I) zugesetzt wird.

6. Hochkonzentrierte Betaine gemäß Anspruch 1, enthaltend 30 bis 50 Gew.-% einer Verbindung der allgemeinen Formel (IV) dadurch gekennzeichnet, daß R³ der Rest der Kokosfettsäure ist.

## Claims

1. Highly concentrated betaines comprising from 30 to 50 % by weight of a compound of the general formula (IV)
R³-CONH-(CH₂)ₘ-N⁺R⁴R⁵(CH₂)_{y}COO⁻ (IV),
wherein R³ is the alkyl radical of a fatty acid, which radical is optionally branched, may optionally contain multiple bonds and may optionally contain hydroxyl groups, R⁴ and R⁵ are identical or different alkyl radicals having 1 - 4 carbon atoms and m may be 1 - 3, from 0.5 to 5 % by weight of at least one compound of the general formula (I) 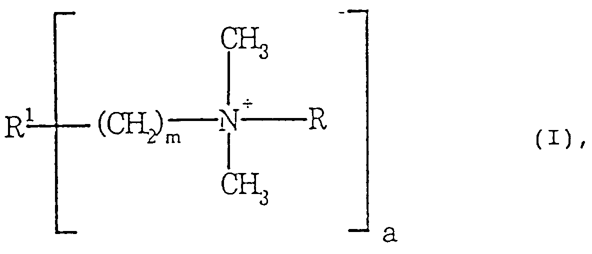 where R = CH₃-, -CH₂COO⁻ K⁺ where K⁺ is ammonium or an alkali metal cation, a = 1 - 2, m = 1 - 3,
R¹ = -N(R)₂ where R = -CH₂COO⁻ K⁺,
R¹ = OH where R = CH₃ and m = 2,
R¹ = -NHCO-(CHR²)ₓ-CH₃ where R² = H or one or more C₁- to C₃-alkyl groups and/or OH groups randomly distributed over the chain and x = from 0 to 8,
R¹ = NHCO-(CHR²)_{y}-CONH- where R² = H or one or more C₁-C₃-alkyl groups randomly distributed over the chain and where a = 2 and y = from 0 to 8,
from 5 to 8 % by weight of sodium chloride and sufficient water to make up to 100 % by weight.

2. Highly concentrated betaines according to claim 1, comprising from 2 to 4 % by weight of the compound of the general formula (I) where R¹ = -N(CH₂COO⁻ K⁺)₂, R = -CH₂COO⁻ K⁺ and m = 3.

3. Highly concentrated betaines according to claim 1, comprising from 2 to 4 % by weight of the compound of the general formula I where R¹ = -NHCO-CH₃ and R = -CH₂COO⁻ K⁺ and m = 3.

4. Highly concentrated betaines according to claim 1, comprising from 2 to 4 % by weight of the compound of the general formula (I) where R¹ = -NHCO-(CH₂)₄-CONH- and m = 3.

5. Process for the production of highly concentrated flowable aqueous solutions of betaines by quaternisation of compounds containing tertiary amine nitrogen using halocarboxylic acids, characterised in that there is added to the reaction mixture, before or during the quaternisation reaction, at least one of the compounds of the general formula (I).

6. Highly concentrated betaines according to claim 1, comprising from 30 to 50 % by weight of a compound of the general formula (IV), characterised in that R³ is the radical of coconut fatty acid.

## Revendications

1. Bétaïnes très concentrées, contenant
30 à 50 % d'un composé de formule générale (IV)
R³-CONH-(CH₂)ₘ-N⁺R⁴R⁵(CH₂)_{y}COO⁻ (IV)
dans laquelle R³ représente le reste alkyle d'un acide gras éventuellement ramifié, qui peut contenir éventuellement des liaisons multiples et éventuellement des groupes hydroxyle, R⁴ et R⁵ représentent des restes alkyle identiques ou différents de 1 à 4 atomes de carbone et m peut être compris entre 1 et 3, 0,5 à 5 % en masse d'au moins un composé de formule générale (I) 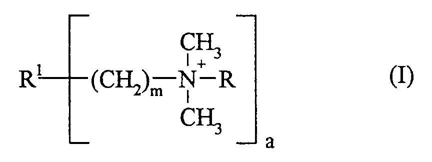
dans laquelle R = CH₃-, -CH₂COO⁻ K⁺, K⁺ étant un cation ammonium ou un cation de métal alcalin;
a = 1-2,
m = 1-3,
R¹ = -N(R)₂ avec R = -CH₂COO⁻ K⁺,
R¹ = OH avec R = CH₃ et m = 2;
R¹ = -NHCO-(CHR₂)ₓ-CH₃ avec R² = H ou un ou plusieurs groupes alkyle en C₁-C₃ et/ou groupes OH répartis de manière statistique sur la chaîne, et x = 0 à 8;
R¹ = -NHCO-(CHR²)_{y}-CONH- avec R² = H ou un ou plusieurs groupes alkyle en C₁-C₃ répartis de manière statistique sur la chaîne, où a = 2 et y = 0 à 8,5 à 8 % en masse de chlorure de sodium et de l'eau pour compléter à 100 % en masse.

2. Bétaïnes très concentrées selon la revendication 1, contenant 2 à 4 % en masse du composé de formule générale (I) dans laquelle R¹ = -N(CH₂COO⁻ K⁺)₂, R = -CH₂COO⁻ K⁺ et m = 3.

3. Bétaïnes très concentrées selon la revendication 1, contenant 2 à 4 % en masse du composé de formule générale (I) dans laquelle R¹ = -NHCO-CH₃ et R = -CH₂COO⁻ K⁺ et m = 3.

4. Bétaïnes très concentrées selon la revendication 1, contenant 2 à 4 % en masse du composé de formule générale (I) dans laquelle R¹ = -NHCO-(CH₂)₄-CONH- et m = 3.

5. Procédé de préparation de solutions aqueuses fluides très concentrées de bétaïnes par quaternisation de composés contenant des atomes d'azote tertiaire avec des acides halogénocarboxyliques, caractérisé en ce que l'on ajoute au mélange réactionnel, avant ou après la réaction de quaternisation, au moins l'un des composés de formule générale (I).

6. Bétaïnes très concentrées selon la revendication 1, contenant 30 à 50 % en masse d'un composé de formule générale (IV), caractérisées en ce que R³ est le reste de l'acide gras de coco.
